Europäisches Patentamt

European Patent Office

Office européen des brevets

(11) Publication number: **0 225 143**
**A2**

# (12) EUROPEAN PATENT APPLICATION

(21) Application number: **86309137.7**

(22) Date of filing: **21.11.86**

(51) Int. Cl.⁴: **C 07 C 29/16**
C 07 C 45/50, C 07 C 85/08,
C 07 C 51/14, C 07 C 51/12,
C 07 C 7/167, C 07 C 5/09,
C 07 C 2/78

(30) Priority: **27.11.85 GB 8529244**

(43) Date of publication of application:
**10.06.87 Bulletin 87/24**

(84) Designated Contracting States:
**BE DE FR GB IT NL**

(71) Applicant: **The British Petroleum Company p.l.c.**
**Britannic House Moor Lane**
**London EC2Y 9BU (GB)**

(72) Inventor: **Brophy, John Howard**
**The British Petroleum Company p.l.c. Chertsey Road**
**Sunbury-on-Thames Middlesex, TW16 7LN (GB)**

**Smith, David John Harry**
**The British Petroleum Company p.l.c. Chertsey Road**
**Sunbury-on-Thames Middlesex, TW16 7LN (GB)**

(74) Representative: **Fawcett, Richard Fennelly et al**
**BP INTERNATIONAL LIMITED Patents Division Chertsey**
**Road**
**Sunbury-on-Thames Middlesex, TW16 7LN (GB)**

(54) The production of ethylene-derived chemicals from gaseous paraffins.

(57) Ethylene-derived chemical products are produced from gaseous paraffinic hydrocarbons by the steps of:

(I) feeding a gaseous paraffinic hydrocarbon, hydrogen and an oxygen-containing gas having a ratio of hydrocarbon to oxygen greater than the stoichiometric ratio for complete combustion to a partial oxidation zone maintained under conditions whereby partial oxidation and simultaneous cracking of the hydrocarbon occurs to form a product comprising acetylene, ethylene, carbon monoxide and hydrogen and recovering the product therefrom,

(II) cooling the product from step (I) to a temperature below 500°C,

(III) contacting under selective hydrogenation conditions the cooled product recovered from step (II) with a catalyst active in the presence of ethylene and carbon monoxide for the selective hydrogenation of acetylene to ethylene to form a product comprising ethylene, carbon monoxide and hydrogen, and

(IV) either (i) separating the product from step (III) into ethylene, carbon monoxide and hydrogen, recovering the carbon monoxide and hydrogen and reacting the ethylene so-separated to form ethylene-derived products, or (ii) reacting ethylene, carbon monoxide, and hydrogen obtained in step (III) without separation to produce ethylene-derived products, or (iii) separating and recovering hydrogen from the product of step (III) and reacting the ethylene and carbon monoxide without further separation to produce ethylene-derived products.

EP 0 225 143 A2

**Description**

## THE PRODUCTION OF ETHYLENE-DERIVED CHEMICALS FROM GASEOUS PARAFFINS

The present invention relates to a process for the production of ethylene-derived chemicals from gaseous paraffinic hydrocarbons.

Until comparatively recently the petrochemicals industry was largely based on feedstocks derived from petroleum. In the 1970's concern regarding the long-term availability and economic viability of petroleum stimulated research into alternative chemical feedstocks such as carbon monoxide and hydrogen, which are derivable from paraffinic hydrocarbons, in particular natural gas of which there are large reserves. In the recently commercialised Monsanto process, methanol derived from carbon monoxide and hydrogen is carbonylated using a rhodium catalyst to produce acetic acid. A desirable objective would be to convert gaseous paraffinic hydrocarbons to chemical products without the intermediate formation of methanol and with the minimum of process steps.

In this connection Japanese Laid-Open Patent Application No. 57-11928 discloses a method of manufacturing ethylene and a gas having hydrogen and carbon monoxide as its main components (synthesis gas feedstock) by partially burning methane using an oxygen-containing gas to produce a gas having acetylene, hydrogen and carbon monoxide as its main components, passing the gas so-produced over a hydrogenation catalyst and semi-hydrogenating acetylene in the gas to give ethylene, separating ethylene from the mixed gas and refining the remainder, so giving pure ethylene and synthesis gas feedstock for chemicals production. A problem attending this process for chemicals production is that the molar ratio of ethylene to carbon monoxide resulting from conventional partial combustion processes using methane/oxygen feeds is low and requires to be higher for both ethylene production and for applications where the ethylene, carbon monoxide and optionally hydrogen are reacted together. Low ethylene/carbon monoxide ratios (very much less than 1) lead to excess carbon monoxide production and possibly also to processing difficulties in homogeneous systems for conversion of ethylene/carbon monoxide/hydrogen mixtures. Desirably the ethylene/carbon monoxide ratio should therefore be as high as possible. Current technology using methane/oxygen feeds limits this ratio to ca. 0.33 assuming 100% acetylene hydrogenation.

A second problem associated with the prior art process is that the only catalyst disclosed for the hydrogenation of acetylene to ethylene is palladium on granular alumina, which in our experience is unsatisfactory for the purpose. We have found that such catalysts are poisoned in low temperature operation by carbon monoxide and in high temperature operation, although carbon monoxide poisoning is less predominant, the selectivity of acetylene hydrogenation to ethylene is drastically reduced by simultaneous hydrogenation to ethane.

We have now developed an improved process for the production of ethylene-derived chemicals from gaseous paraffinic hydrocarbons which process substantially overcomes the problems associated with the prior art process.

Accordingly the present invention provides a process for the production of ethylene-derived chemical products from gaseous paraffinic hydrocarbons which process comprises the steps of:

(I) feeding a gaseous paraffinic hydrocarbon, hydrogen and an oxygen-containing gas having a ratio of hydrocarbon to oxygen greater than the stoichiometric ratio for complete combustion to a partial oxidation zone maintained under conditions whereby partial oxidation and simultaneous cracking of the hydrocarbon occurs to form a product comprising acetylene, ethylene, carbon monoxide and hydrogen and recovering the product therefrom,

(II) cooling the product from step (I) to a temperature below 500°C,

(III) contacting under selective hydrogenation conditions the cooled product recovered from step (II) with a catalyst active in the presence of ethylene and carbon monoxide for the selective hydrogenation of acetylene to ethylene to form a product comprising ethylene, carbon monoxide and hydrogen, and

(IV) Either (i) separating the product from step (III) into ethylene, carbon monoxide and hydrogen, recovering the carbon monoxide and hydrogen, and reacting the ethylene so-separated to form ethylene-derived products, or (ii) reacting ethylene, carbon monoxide, and hydrogen obtained in step (III) without separation to produce ethylene-derived products, or (iii) separating and recovering hydrogen from the product of step (III) and reacting the ethylene and carbon monoxide without further separation to produce ethylene-derived products.

The gaseous paraffinic hydrocarbon may suitably be substantially pure methane or ethane or mixtures of hydrocarbons comprising substantial proportions of methane and/or ethane, for example mixtures of hydrocarbons obtained from natural hydrocarbon gas reservoirs, which may also contain carbon dioxide. The preferred gaseous paraffinic hydrocarbon is either methane or natural gas. The oxygen-containing gas may be, for example, air or an air/oxygen mixture. It is preferred, however, to use substantially pure oxygen as the oxygen-containing gas.

By co-feeding hydrogen to the partial oxidation zone using methane feed the ethylene/carbon monoxide ratio in the subsequent selectively hydrogenated product can be increased to a value of ca. 0.7, thereby facilitating its subsequent conversion into ethylene-derived products and increasing the yield of these products whilst simultaneously decreasing the yield of unconverted carbon monoxide.

Any process which produces a mixture of acetylene, ethylene, carbon monoxide and hydrogen from saturated hydrocarbons and hydrogen by simultaneous partial oxidation and cracking of the hydrocarbon may be utilised as step (I) of the process of the invention.

A suitable process is described in our copending International application no. PCT/GB 86/00198 (BP Case No. 6042), reference to which may be made for further details.

Another suitable process is described in our copending UK application No. 8607559 (BP Case No. 6350), the disclosure of which is incorporated by reference herein.

In a particularly preferred embodiment of the present invention step (I) comprises the process described in our copending European application publication No. 0178853 (BP Case No. 5959), which discloses a process for the production of synthesis gas and hydrocarbons in which (a) a saturated hydrocarbon and an oxygen-containing gas having a ratio of hydrocarbon to oxygen of greater than the stoichiometric ratio for complete combustion are introduced together with hydrogen into a bed of particulate material, (b) the upward flow rate of the gases being sufficiently large to fluidise or to cause a spouting action of the bed material (c) the hydrocarbon. oxygen-containing gas and hydrogen being ignited and reacted together and (d) the products of the reaction being withdrawn. For a detailed description of this process the reader is referred to the aforesaid European patent publication.

In step (II) of the process of the invention the product from step (I), which may typically be at a temperature in the range from ca 800°C to ca 1000°C, is cooled to a temperature below 500°C. The cooling may be effected in conventional manner, for example by heat-exchange or by quenching. Quenching may suitably be accomplished by a water quench or a hydrocarbon quench. It is preferred to cool the product of step (I) by quenching with a liquid hydrocarbon crackable to ethylene, thereby beneficially using the heat content of the hot product. The temperature to which the product is cooled is preferably that temperature which is optimium for the operation of step (III).

In step (III) of the process of the present invention the cooled product comprising acetylene, ethylene, carbon monoxide and hydrogen recovered in step (II) is contacted under selective hydrogenation conditions with a catalyst active in the presence of carbon monoxide and ethylene for the selective hydrogenation of acetylene to ethylene to form a product comprising ethylene, carbon monoxide and hydrogen.

Suitable selective hydrogenation catalysts comprise an oxide or a sulphide of a metal or mixture of metals having hydrogenation activity, as described in our copending UK application No. 8529245 (BP Case No. 6249). Suitable metals having hydrogenation activity, which in the form of their oxides or sulphides, may be used as the selective hydrogenation catalyst in the process of the present invention include, for example, zinc, gallium, copper, cadmium, chromium. molybdenum, tungsten, cobalt, nickel, ruthenium and iron, of which zinc is preferred, either alone or mixed with at least one of the aforesaid metals.

Catalyst compositions comprising more than one oxide may suitably be prepared by mixing the individual oxides, or compounds thermally decomposable to the oxides, for example by mixing a suspension of the compounds in water and evaporating the mixture to form a cake. Precipitation or coprecipitation may also be employed. The cake may thereafter be filtered, washed and dried and the dried cake crushed and calcined at elevated temperature to produce the desired catalyst composition. The calcination may suitably be carried out in an oxidising atmosphere, for example in air.

The catalysts may, if desired, be subjected to a suitable pretreatment. The pretreatment may take the form of heating in air at elevated temperature or heating in nitrogen or hydrogen or incorporating an effective amount of an alkali metal, for example potassium.

Examples of catalysts suitable for use in the selective hydrogenation step include $ZnO$, $CR_2O_3/ZnO$ and $ThO_2/ZnO/Ga_2O_3$. Suitable catalysts of the $ThO_2/ZnO/Ga_2O_3$ - type are described in our European application publication No. 0070690, to which the reader is referred for further details. These catalysts are more tolerant to poisoning and are more selective for the hydrogenation of acetylene to ethylene than the prior art palladium/alumina catalyst.

Selective hydrogenation conditions using the catalysts as aforesaid for example may suitably be an elevated temperature in the range from 100 to 500°C, generally greater than 250°C. The optimum temperature within the aforesaid range will depend on the nature of the catalyst selected. The pressure may suitably be in the range from 1 to 50 bars. The GHSV may suitably be in the range from 100 to 10,000 $h^{-1}$.

In addition to the hydrogen produced in step (I) of the process, further hydrogen may if desired be added in step (III).

Whilst it is preferred to operate step (III) by contacting a gaseous feedstock with the hydrogenation catalyst in the form of a fixed bed, a moving bed or a fluidised bed, it may be operated in other ways, for example by contacting a gaseous feedstock with a slurry of the catalyst in a suitable solvent.

In alternative (i) of step (IV) of the process of the present invention the product from step (III) is separated into ethylene, carbon monoxide and hydrogen, the ethylene is reacted to form ethylene-derived products and the hydrogen and carbon monoxide are recovered. Ethylene may be separated from carbon monoxide and hydrogen by means well-known in the art. Ethylene so-obtained may be used as feedstock for any process converting ethylene to products, such as for example the production of polyethylene, which may be of the low-density, high-density or linear low-density type.

The recovered carbon monoxide and hydrogen may be used as feedstock for example for the production of methanol, for the production of mixed lower aliphatic alcohols or for the production of hydrocarbons and/or oxygenated derivatives thereof. Catalysts and processes for effecting such conversions are well-known in the art.

In alternative (ii) of step (IV), ethylene, carbon monoxide and hydrogen obtained in step (III) are reacted without further separation to produce ethylene-derived products. This is a preferred method of operating step (IV). Processes in which the product from step (IV) may be used without any intervening process steps, other than possibly the addition of another reactant, possible adjustment of the proportions of the individual components and possible adjustment of the temperature and pressure include:

(a) the rhodium or cobalt-catalysed hydroformylation of ethylene to propionaldehyde according to the equation:

$$CH_2 = CH_2 + CO + H_2 \rightarrow CH_3CH_2CHO$$

This process is described in, for example, US Patent No. 1,599,922 to which reference may be made for further details.

(b) the cobalt-catalysed hydroformylation of ethylene to propanol according to the equation:

$$CH_2 = CH_2 + CO + 2H_2 \rightarrow CH_3CH_2CH_2OH$$

This process is described in, for example, US Patent No. 3,239,571 to which reference may be made for further details.

(c) the production of diethyl ketone from ethylene, carbon monoxide and hydrogen in the presence of a palladium/phosphine catalyst according to the equation:

$$2CH_2 = CH_2 + CO + H_2 \rightarrow C_2H_5COC_2H_5$$

This process is described for example by V.N. Zudin, G.N. Il'inich, V.A. Likholobov and Y.I. Yermakov in J. Chem. Soc. Chem. Commun., 1984, 545.

(d) the production of amines from ethylene according to the equation:

$$CH_2 = CH_2 + R_2NH + CO + 2H_2 \rightarrow R_2NPr + H_2O$$

This process is described by R.A. Sheldon in Chemicals from Synthesis Gas, D. Reidel Dordrecht, 1983, p 100 to which reference may be made for further details.

In alternative (iii) of step (IV), ethylene and carbon monoxide without further separation are reacted to produce ethylene-derived products. Hydrogen may be separated from the product of step (III) by methods known in the art, for example by membrane separation or by pressure swing adsorption. Processes in which ethylene and carbon monoxide may be used without any intervening process steps, other than possibly the addition of another reactant, possible adjustment of the relative proportions of the individual components or possible adjustment of the temperature and/or pressure include:

(e) the hydrocarboxylation of ethylene in the presence of methanol to co-produce acetic and propionic acids according to the equations:

$$CH_2 = CH_2 + CO + H_2 \rightarrow CH_3CH_2CO_2H$$
$$CH_3OH + CO \rightarrow CH_3CO_2H$$

This process is described in, for example, US Patent No. 4,111,982 to which reference may be made for further details.

(f) the hydrocarboxylation of ethylene to propionic acid according to the equation:

$$CH_2 = CH_2 + CO + H_2O \rightarrow CH_3CH_2CO_2H$$

This process is described by G.W. Parshall in Homogeneous Catalysis published in 1980 by Wiley-Interscience, pages 82 to 85, to which reference may be made for further details.

(g) the reductive carbonylation of ethylene to diethyl ketone according to the equation:

$$2CH_2 = CH_2 + 2CO + H_2O \rightarrow CH_3CH_2 \overset{\text{O}}{\underset{\|}{C}} CH_2CH_3 + CO_2$$

This process is described by Murata et al, Bull Chem. Soc. Japan, 1981, 2089-92, to which reference may be made for further details.

(h) the production of dialkylpropanamides according to the equation:

$$CH_2 = CH_2 + CO + HNR_2 \rightarrow C_2H_5CONR_2$$

wherein R is alkyl, preferably lower alkyl, for example ethyl. In contrast to processes (a) to (d) wherein hydrogen is an essential reactant, for the purpose of processes (e) to (h) substantially all the hydrogen must be removed from the product of step (II) before the reductive carbonylation reaction.

The process of the present invention will now be further illustrated by reference to the following Examples.

Step (I)

Step (I) was carried out using the method and apparatus disclosed in the example of our copending European application publication No. 0178853 (BP Case No. 5959) to produce gaseous compositions principally comprising hydrogen, carbon monoxide, ethylene and acetylene as shown in Tables 1 and 2 of the aforesaid European patent publication.

Step (III)

Examples A to D demonstrate that a feed simulating in composition the product of Step (I) and comprising acetylene, ethylene, carbon monoxide and hydrogen can be reacted in the presence of a hydrogenation catalyst to selectively hydrogenate the acetylene in the feed to ethylene.

Comparison Tests E to H are included to show that the prior art palladium/alumina catalyst is inferior to the metal oxide catalysts as used in Examples A to D, which are preferred catalysts in the process of the present invention.

Example A

A gaseous feed consisting of acetylene (6.4%), ethylene (3.2%), carbon monoxide (29%) and hydrogen (61.4%), all percentages being expressed by volume, was contacted at 3 bars pressure and at a GHSV of 1000 $h^{-1}$ with a $ThO_2/ZnO/Ga_2O_3$ catalyst maintained at a temperature of 377°C for a period of 2 hours. The acetylene conversion was 100% and the selectivities (% C-mol) were:

$CO_2$ - 9
$CH_4$ - 1
$C_2H_4$ - 56
$C_2H_6$ - 3
$C_3+$ - 31

Example B

Example A was repeated except that instead of the $ThO_2/ZnO/Ga_2O_3$ catalyst there was used a $Cr_2O_3/ZnO$ catalyst.

The acetylene conversion was 100% and the selectivities (% C-mol) were:

$CO_2$ - 1
$CH_4$ - 2
$C_2H_4$ - 56
$C_2H_6$ - 6
$C_3+$ - 35

Example C

Example A was repeated except that instead of the $ThO_2/ZnO/Ga_2O_3$ catalyst there was used a ZnO catalyst and the GHSV was reduced to 985 $h^{-1}$

The acetylene conversion was 100% and the selectivities (% C-mol) were:

$CO_2$ - 1
$CH_4$ - 1
$C_2H_4$ - 79
$C_2H_6$ - 3
$C_3+$ - 16

Example D

Example C was repeated except that the temperature was reduced to 327°C and the GHSV was increased to 1465 $h^{-1}$.

The acetylene conversion was 100% and the selectivities (% C-mol) were:

$CO_2$ - none
$CH_4$ - 1
$C_2H_4$ - 71
$C_2H_6$ - 2
$C_3+$ - 26

Comparison Test E

A gaseous feed consisting of acetylene (6.4% by volume), ethylene (3.2% by volume), carbon monoxide (29% by volume) and hydrogen (61.4% by volume) was contacted at a GHSV of 870 $h^{-1}$ with a commercially available palladium supported on alumina catalyst maintained at a temperature of 115°C (the recommended operating temperature) operated at 1 bar pressure for a period of 1 hour.

The conditions and results obtained are given in Table 1.

Comparison Test F

Comparison Test E was repeated except that the feed was contacted with the catalyst for a period of 2 hours.

The conditions and the results obtained are given in Table 1.

Comparison Tests E and F are included to show that the prior art acetylene hydrogenation catalyst is rapidly poisoned by carbon monoxide in the feed at low temperatures.

Comparison Test G

Comparison Test F was repeated except that the temperature was raised to 177°C, the GHSV reduced to 293 $h^{-1}$ and the pressure raised to 3 bar.

The conditions and results obtained are given in Table 1.

Comparison Test H

Comparison Test G was repeated except that the temperature was raised to 377°C and the GHSV was increased to 1010 $h^{-1}$.

The conditions and results obtained are given in Table 1.

Comparison Tests G and H are included for the purpose of demonstrating that at elevated temperature the

5

prior art acetylene hydrogenation catalyst is very active for hydrogenation, but is unselective, the acetylene being totally hydrogenated to ethane.

TABLE 1

|  | Comparative Tests | | | |
|  | E | F | G | H |
|---|---|---|---|---|
| Catalyst | Pd/Al$_2$O$_3$ (G63) | | | |
| GHSV/h$^{-1}$ | 870 | 870 | 293 | 1010 |
| Temp/°C | 115 | 115 | 177 | 377 |
| Pressure/bar | 1 | 1 | 3 | 3 |
| Hours-on-Stream | 1 | 2 | 2 | 2 |
| C$_2$H$_2$ Conversion | 57 | 17 | 100 | 100 |
| Selectivities % C-mol | | | | |
| CO$_2$ | – | – | – | 3 |
| CH$_4$ | – | – | – | 17 |
| C$_2$H$_4$ | 91 | 78 | – | – |
| C$_2$H$_6$ | – | 7 | 95 | 73 |
| C$_3^+$ | 9 | 15 | 5 | 7 |

Feed Composition (% vol)

$C_2H_2$ = 6.4%

$C_2H_4$ = 3.2%

CO = 29%

$H_2$ = 61.4%

Step (IV)

In Examples 1 to 4 and 6 a feed simulating a composition obtainable from step (III) of the process of the invention by selective hydrogenation of the product of step (II) was employed. In Example 5 and 7 hydrogen was omitted from the feed.

Example 1 - Rhodium catalysed hydroformylation of ethylene to propionaldehyde

RhH(CO)(PPh$_3$)3 (0.028g) and triphenylphosphine (0.4g) were added to 1-pentanol (60 mls) in a 300 ml stainless steel autoclave, and the system flushed three times (at a pressure of 30 bars) with 2:1 H$_2$:CO. The autoclave was then charged with a mixture of ethylene/CO/H$_2$ (1:3:6) to a pressure of 45 bar, and heated to 85°C for 1 h. The vessel was cooled to room temperature and a pressure of 32 bar. The reaction mixture was analysed by gc and shown to contain 5.5% w/w propanal and 0.05% w/w propanol.

Example 2 - Cobalt catalysed hydroformylation of ethylene to propanol

·Co$_2$(CO)$_8$ (0.0539g) and PBu$^n_3$ (0.110 mls) were quickly added to 1-pentanol (60mls) in a 300 ml stainless steel autoclave and the system flushed three times (at a pressure of 30 bar) with 2:1 H$_2$:CO. The autoclave was then charged with a mixture of ethylene/CO/H$_2$ (1:3:6) to a pressure of 57 bar at room temperature. The contents of the autoclave were heated to 180°C and 77 bar for 2h. Upon cooling to room temperature the pressure dropped to 41 bar. The reaction mixture was then analysed by gc and found to contain 0.8% w/w propanal, 4.0% w/w propanol.

Example 3 - Hydrocarboxylation of ethylene in the presence of methanol

Acetic acid (71 mls), water (20 mls), methanol (3 mls) and methyl iodide (4.04 mls) were all charged to a 300 ml corrosion-resistant autoclave equipped with magnedrive stirrer, thermocouple and cooling coils; in addition, a liquid injection mechanism with a dip tube was fitted to the autoclave. The autoclave was flushed three times with nitrogen, then charged with the same gas to a pressure of 7 bar. The autoclave and contents were heated to a temperature of 165°C. A solution of [RhCl(CO)$_2$]$_2$ (0.065 g) in acetic acid (9 mls) was charged to the autoclave via the liquid injection port and the overall pressure adjusted to 35 bar with a 1:1 ethylene/carbon monoxide mixture. The temperature was maintained at 165°C for 1 h and the ethylene/carbon monoxide fed at such a rate as to maintain a pressure of 35 bar in the reactor. The reactor was then cooled to ambient temperature. Analysis of the reaction products by g.c. indicated the presence of methyl acetate (1.33 x 10⁻³ moles), ethyl iodide ($1.11 \times 10^{-2}$ moles), ethyl acetate ($7.37 \times 10^{-4}$ moles), acetone ($4.04 \times 10^{-3}$ moles), propionic acid ($6.05 \times 10^{-1}$ moles) and product acetic acid ($8.61 \times 10^{-2}$ moles.)

Example 4 - Hydrocarboxylation of ethylene to propionic acid

Example 3 was repeated but no methanol was charged to the autoclave. Analysis of the reaction products indicated the presence of acetone ($2.5 \times 10^{-3}$ moles), methyl acetate ($7.0 \times 10^{-4}$ moles), ethyl iodide ($1.86 \times 10^{-2}$ moles), ethyl acetate ($7.0 \times 10^{-4}$ moles), and propionic acid ($4.27 \times 10^{-1}$ moles).

Example 5 - Hydroamidation of ethylene to propanamides

Cobalt octacarbonyl (3.0 mmol), diethylamine (131 mmol) and hexanol (60 cm³) were placed in a 300 cm³ stainless steel autoclave fitted with a magnedrive stirrer, thermocouple and cooling coil. This was then purged with carbon monoxide and charged with 15 bar (150 mmol) ethylene and 125 bar CO. The reaction mixture was heated to 200°C and maintained at this temperature for 2 hours. The autoclave was subsequently cooled to room temperature and vented. Gas chromatography of the liquid products showed a 85.5% conversion of diethylamine to N,N-diethylpropanamide together with smaller amounts of N-ethyl-propanamide and diethylformamide.

Example 6 - Formation of amines

Into a 300 cm³ stainless steel autoclave fitted with a magnedrive stirrer, thermocouple and cooling coil were placed cobalt octacarbonyl (3.0 mmol), diethylamine (148 mmol) and hexanol (60 cm³). This autoclave was pressurised to 220 bar with a 6:3:1 mole ratio mixture of hydrogen, carbon monoxide and ethylene and heated to 210°C and a total pressure of 300 bar. As gas was taken up by the reaction the pressure was maintained at 280 bar by addition of the gas mixture. After 2h the autoclave was cooled to room temperature and vented. Analysis of the liquid products by gas chromatography showed 65.9% conversion of diethylamine with selectivities of 39.8% to N,N-diethylpropylamine, 10.3% to diethylformamide, 9.3% to N-ethylpropylamine, 6.6% to triethylamine and 4.0% to N,N-diethylpropanamide.

Example 7 - Reductive carbonylation of ethylene to diethyl ketone

Co₂(CO)₈ (1.473g), diphos (1.72g) and water (2.6 mls) were all added to deoxygenated 1,4-dioxan (100g) in a nitrogen flushed 300 ml stainless steel autoclave. The system was flushed twice with carbon monoxide at a pressure of 20 bar. The autoclave was then charged with a 3:1 CO: ethylene mixture to a pressure of 160 bar. The autoclave was heated to 165°C and maintained at this temperature for 140 minutes. The autoclave was cooled to ambient temperature (and a pressure of 82 bar). The contents were analysed by gc and found to contain 10.9% w/w diethyl ketone.

Example 8 - The production of diethyl ketone from ethylene, carbon monoxide and hydrogen

Palladium acetate (2.2 mmol), triphenylphosphine (15.0 mmol), trifluoroacetic acid (57 cm³), and water (3 cm³) were placed in a 300 cm³ Hastelloy autoclave. This was then purged with carbon monoxide, charged to 30 bar with a 2:1:1 $C_2H_4/CO/H_2$ mixture and heated to 65°C for 15 minutes, the pressure being restored to 30 bar after 5 minutes by addition of a further 20 bar of gas mixture. After the reaction period the autoclave was cooled and vented. Gas chromatography of both the gaseous and liquid phases indicated that over 95% of the ethylene had reacted with selectivities of 70.7% to diethyl ketone, 10.3% to $EtCOCH_2CH_2COEt$, 5.5% to propionic acid, and 9.1% to higher ketones.

## Claims

1 A process for the production of ethylene-derived chemical products from gaseous paraffinic hydrocarbons which process comprises the steps of:

(I) feeding a gaseous paraffinic hydrocarbon, hydrogen and an oxygen-containing gas having a ratio of hydrocarbon to oxygen greater than the stoichiometric ratio for complete combustion to a partial oxidation zone maintained under conditions whereby partial oxidation and simultaneous cracking of the hydrocarbon occurs to form a product comprising acetylene, ethylene, carbon monoxide and hydrogen and recovering the product therefrom,

(II) cooling the product from step (I) to a temperature below 500°C,

(III) contacting under selective hydrogenation conditions the cooled product recovered from step (II) with a catalyst active in the presence of ethylene and carbon monoxide for the selective hydrogenation of acetylene to ethylene to form the product comprising ethylene, carbon monoxide and hydrogen, and

(IV) either (i) separating the product from step (III) into ethylene, carbon monoxide and hydrogen, recovering the carbon monoxide and hydrogen and reacting the ethylene so-separated to form ethylene-derived products, or (ii) reacting ethylene, carbon monoxide, and hydrogen obtained in step (III) without separation to produce ethylene-derived products, or (iii) separating and recovering hydrogen from the product of step (III) and reacting the ethylene and carbon monoxide without further separation to produce ethylene-derived products.

2 A process according to claim 1 wherein the gaseous paraffinic hydrocarbon is methane.

3 A process according to claim 1 wherein the gaseous paraffinic hydrocarbon is natural gas.

4 A process according to claim 1 wherein the oxygen-containing gas is oxygen.

5 A process according to any one of the preceding claims wherein step (I) comprises a process in which (a) a saturated hydrocarbon and an oxygen-containing gas having a ratio of hydrocarbon to oxygen of greater than the stoichiometric ratio for complete combustion are introduced together with hydrogen into a bed or particulate material, (b) the upward flow rate of the gases being sufficiently large to fluidise or to cause a spouting action of the bed material (c) the hydrocarbon, oxygen-containing gas and hydrogen being ignited and reacted together and (d) the products of the reaction being withdrawn.

6 A process according to any one of the preceding claims wherein the selective hydrogenation catalyst of step (III) comprises an oxide or a sulphide of a metal or mixture of metals having hydrogenation activity.

7 A process according to claim 6 wherein the metal is zinc either alone or in combination with at least one other metal.

8 A process according to either claim 6 or claim 7 wherein the catalyst is either zinc (II) oxide (ZnO), zinc (II) oxide (ZnO)/chromium (III) oxide ($Cr_2O_3$) or zinc (II) oxide (ZnO)/thorium (IV) oxide ($ThO_2$)/gallium (III) oxide ($Ga_2O_3$).

9 A process according to any one of the preceding claims wherein step (IV) is alternative (i) and the separated ethylene is converted to polyethylene.

10 A process according to any one of claims 1 to 8 wherein step (IV) is alternative (ii) and the ethylene, carbon monoxide and hydrogen are reacted either:

    (a) in the presence of a rhodium or a cobalt catalyst under hydroformylation conditions to produce propionaldehyde, or

    (b) in the presence of a cobalt catalyst under hydroformylation conditions to produce propanol, or

    (c) in the presence of a palladium/phosphine catalyst to produce diethylketone, or

    (d) in the presence of a secondary amine to produce a tertiary amine.

11 A process according to any one of claims 1 to 8 wherein step (IV) is alternative (iii) and the ethylene and carbon monoxide are reacted either:

    (a) in the presence of methanol and water to produce a mixture of acetic and propionic acids, or

    (b) in the presence of water to produce propionic acid, or

    (c) in the presence of water to produce diethyl ketone, or

    (d) in the presence of a secondary amine to produce dialkylpropanamides.